# EUROPEAN PATENT APPLICATION

(11) **EP 3 722 813 A1**
(43) Date of publication of application: **14.10.2020**
(21) Application number: 18886588.5
(22) Date of filing: 09.10.2018
(51) Int. Cl.: G01N 35/02, B03C 1/00, B03C 1/28, G01N 1/28

(54) **MAGNETIC SEPARATION METHOD AND AUTOMATED ANALYZER**

(30) Priority: 07.12.2017 JP 2017235298
(71) Applicant: Hitachi High-Tech Corporation, Minato-ku Tokyo 105-6409 (JP)
(72) Inventor: HSU, Ruochi, Tokyo 100-8280 (JP); INABA, Tooru, Tokyo 100-8280 (JP); MATSUOKA, Shinya, Tokyo 105-8717 (JP); EBIHARA, Daisuke, Tokyo 105-8717 (JP); YOKOKAWA, Takeshi, Tokyo 105-8717 (JP)
(74) Representative: Strehl Schübel-Hopf & Partner
(86) International application number: PCT/JP2018/037621
(87) International publication number: WO 2019/111524

(57) **Abstract**

The purpose of the present invention is to use a single device to highly efficiently carry out a plurality of washing processes for gradually reducing the amount of a magnetic particle solution in a reaction vessel. This magnetic separation method comprises a plurality of washing processes for using a magnetic separation device and a stirring mechanism to separate a magnetic substance and nonmagnetic substance. A first washing process includes a step for inserting a reaction vessel 116 into a recess provided in the magnetic separation device and capturing the magnetic substance using a plurality of magnets 51, 52 that are each disposed along the peripheral direction of the recess such that the same pole faces the reaction vessel 116, a step for solution aspiration, a step for discharging liquid such that the surface 61 of the liquid goes to a position higher than the upper edges of the magnets, and a step for stirring the liquid. A second washing process includes a step for inserting the reaction vessel 116 into the magnetic separation device and aspirating the liquid, a step for discharging the liquid such that the surface 62 of the liquid goes to a position lower than the upper edges of the magnets where the magnetic field intensity is lower than at the position of the upper edges of the magnets, and a step for stirring the liquid. (See fig. 6.)

## Description

### Technical Field

The present disclosure relates to a magnetic separation method and an automated analyzer for separating a substance to be measured from coexisting substances using magnetic beads.

### Background Art

In analyzing a liquid sample derived from a biological body such as blood or urine with high sensitivity, a technique for selectively identifying a substance to be measured from a sample containing a large amount of coexisting substances is essential. As such a technique, a labeled antibody method for separating the substance to be measured from the sample using magnetic beads is known.

In the above labeled antibody method, the magnetic beads to which a non-labeled antibody that performs an antigen-antibody reaction with the substance to be measured is bound and a labeled antibody labeled with a labeling substance are included in the sample, and a target substance to be measured is bound to the magnetic beads and the labeling substance. Then, the magnetic beads are magnetically separated from the sample to remove the coexisting substances, the substance to be measured is eluted from the magnetic beads, and a content of the target substance can be measured by photometry of the labeling substance.

In an automated analyzer that can carry out the above series of processes, a concentration of the substance to be measured may be increased in order to improve a sensitivity of a measurement. For example, the substance to be measured is bound to the magnetic beads, a washing process is carried out to remove the coexisting substances by capturing the magnetic beads by magnetic separation and aspirating a reaction solution, the substance to be measured is eluted with a relatively small amount of liquid in an elution process, and a high sensitivity measurement is performed by increasing the concentration of the substance to be measured. Further, in the washing process, the magnetic separation and stirring are performed while gradually reducing an amount of a washing liquid to be injected, so that the magnetic beads are prevented from remaining on a reaction vessel wall surface.

PTL 1 discloses a method in which a plurality of magnets are provided in a longitudinal direction to reduce an amount of the magnetic beads flowing out due to a washing operation in a Bound/Free separation (BF separation, a separation of an antigen-antibody conjugate and a non-conjugate) process involving a pre-magnetization and a main magnetism.

PTL 2 describes a technology in which a magnetic force of a magnet provided on an aspirate and discharge system side of a pipette tip or the like of a dispenser is used to adsorb a magnetic body in a short time and with almost perfect accuracy.

PTL 3 discloses an automated analyzer including a unit configured to increase a liquid amount in a reaction vessel before a reaction solution discharging process in a magnetic separation process, and describes that a series of processes of injecting a buffer solution, capturing magnetic beads, and discharging a reaction solution may be performed a plurality of times as necessary.

### Citation list

### Patent Literature

PTL 1: JP-A-2016-085093
PTL 2: JP-A-H8-062224
PTL 3: JP-A-2014-122826

### Summary of Invention

### Technical Problem

However, in the method described in PTL 1, a relationship between a magnet height and the liquid amount of the washing liquid in a two-stage BF separation process is not considered. Therefore, in a case where a surface height of a liquid when injecting the washing liquid in the washing process matches a position where a strong magnetic field where the magnetic beads easily aggregate is generated, it is possible that the magnetic beads aggregate near the surface of the liquid, resulting in poor washing efficiency. In other words, when a plurality of washing processes are performed with the same magnetic separation device while the liquid amount of the washing liquid is reduced, the magnetic beads may aggregate on the vessel wall surface near the surface of the liquid. In a state where the magnetic beads are excessively aggregated, it is difficult to separate impurities which are non-magnetic components, which causes a reduction in washing efficiency. For the above reasons, the washing process in which the liquid amount changes requires using different magnetic separation devices according to the liquid amount, and an operation process is complicated.

Further, in the method described in PTL 2, when the amount of the washing liquid to be used is reduced, the same pipette tip is used, so that there is a possibility that washing cannot be performed sufficiently. On the other hand, when the pipette tip having a plurality of diameters is used in order to cope with a problem of insufficient washing, labor and cost are greatly increased.

Furthermore, in the method described in PTL 3, when the buffer solution is increased, a large amount of the buffer solution is used, and the cost is increased.

The present disclosure has been made in view of the above circumstances, and provides a technology that can use a single device to highly efficiently carry out a plurality of washing processes for gradually reducing a liquid amount of a magnetic bead solution in a reaction vessel.

### Solution to Problem

In order to solve the above problems, the present disclosure provides a magnetic separation method including a plurality of washing processes for separating a magnetic substance and a nonmagnetic substance using a magnetic separation device and a stirring mechanism, in which the plurality of washing processes includes at least a first washing process and a second washing process, the first washing process includes: a step of inserting a reaction vessel containing a solution including the magnetic substance and the nonmagnetic substance into a recess provided in the magnetic separation device and capturing the magnetic substance using a plurality of magnets that are each disposed along a peripheral direction of the recess such that the same pole faces the reaction vessel; a step of aspirating the solution with the magnetic substance being captured; a step of discharging liquid to the reaction vessel such that a surface of the liquid goes to a position higher than upper edges of the magnets; and a step of removing the reaction vessel from the magnetic separation device and stirring the liquid held by the reaction vessel using the stirring mechanism, and the second washing process includes: a step of inserting the reaction vessel into the magnetic separation device and aspirating the liquid with the magnetic substance being captured; a step of discharging the liquid to the reaction vessel such that a surface of the liquid goes to a position lower than the upper edges of the magnets where magnetic field intensity is lower than that at a position of the upper edges of the magnets; and a step of removing the reaction vessel from the magnetic separation device and stirring the liquid held by the reaction vessel using the stirring mechanism.

In addition, the present disclosure provides a magnetic separation method including a plurality of washing processes for separating a magnetic substance and a nonmagnetic substance using a magnetic separation device and a stirring mechanism, in which the plurality of washing processes includes at least a first washing process and a second washing process, the first washing process includes: a step of inserting a reaction vessel containing a solution including the magnetic substance and the nonmagnetic substance into a recess provided in the magnetic separation device and capturing the magnetic substance using a plurality of magnets, the magnets being disposed such that a first stage and a second stage positioned below the first stage along a vertical direction of the recess are each provided with an equal number of magnets, the magnets in the first stage and the magnets in the second stage are vertically adjacent to each other with different poles, two adjacent magnets in the first stage have poles different from each other facing the reaction vessel, and two magnets facing each other have the same pole facing the reaction vessel; a step of aspirating the solution with the magnetic substance being captured; a step of discharging liquid to the reaction vessel such that a surface of the liquid goes to a position higher than upper edges of the magnets; and a step of removing the reaction vessel from the magnetic separation device and stirring the liquid held by the reaction vessel using the stirring mechanism, and the second washing process includes: a step of inserting the reaction vessel into the magnetic separation device and aspirating the liquid with the magnetic substance being captured; a step of discharging liquid to the reaction vessel such that a surface of the liquid goes to a position lower than a position higher than the upper edges of the magnets in the first stage where magnetic field intensity is lower as compared with at a center of the magnets in the first stage or the magnets in the second stage; and a step of removing the reaction vessel from the magnetic separation device and stirring the liquid held by the reaction vessel using the stirring mechanism.

### Advantageous Effect

According to the present disclosure, the plurality of washing processes for gradually reducing the liquid amount of the magnetic bead solution in the reaction vessel can be highly efficiently carried out using the single device. Problems, configurations, and effects other than those described above will be further clarified with the following description of embodiments.

### Brief Description of Drawings

[FIG. 1] FIG. 1 is a schematic diagram of an automated analyzer 1 according to the present disclosure.
[FIG. 2] FIG. 2 is a schematic diagram showing a flow of a process for extracting a substance to be measured contained in a sample.
[FIG. 3] FIG. 3 is a diagram showing a first washing process.
[FIG. 4] FIG. 4 is a schematic diagram showing a flow of an elution process.
[FIG. 5] FIG. 5 shows an example of a magnetic separation device according to the present embodiment.
[FIG. 6] FIG. 6 shows states of capturing magnetic beads in the magnetic separation device.
[FIG. 7] FIG. 7 shows a magnetic separation device according to a second embodiment of the present disclosure.
[FIG. 8] FIG. 8 shows states of capturing magnetic beads in the magnetic separation device according to the second embodiment.
[FIG. 9] FIG. 9 shows magnet arrangements according to a third embodiment.

### Description of Embodiments

Hereinafter, embodiments of the present disclosure will be described with reference to the drawings. The embodiments of the present disclosure are not limited to the embodiments to be described below, and various modifications can be made within the scope of the technical idea thereof. Corresponding parts of the drawings used in the description of each embodiment to be described below are denoted by the same reference numerals, and a repetitive description will be omitted.

Although the embodiments of the present disclosure are mainly directed to an immunoassay analyzer, the present disclosure is applicable to all automated analyzers. The present disclosure can also be applied to, for example, an automated clinical analyzer, a gene analyzer, a mass spectrometer, and a bacteria test device.

### <First Embodiment

### [Configuration of Automated Analyzer]

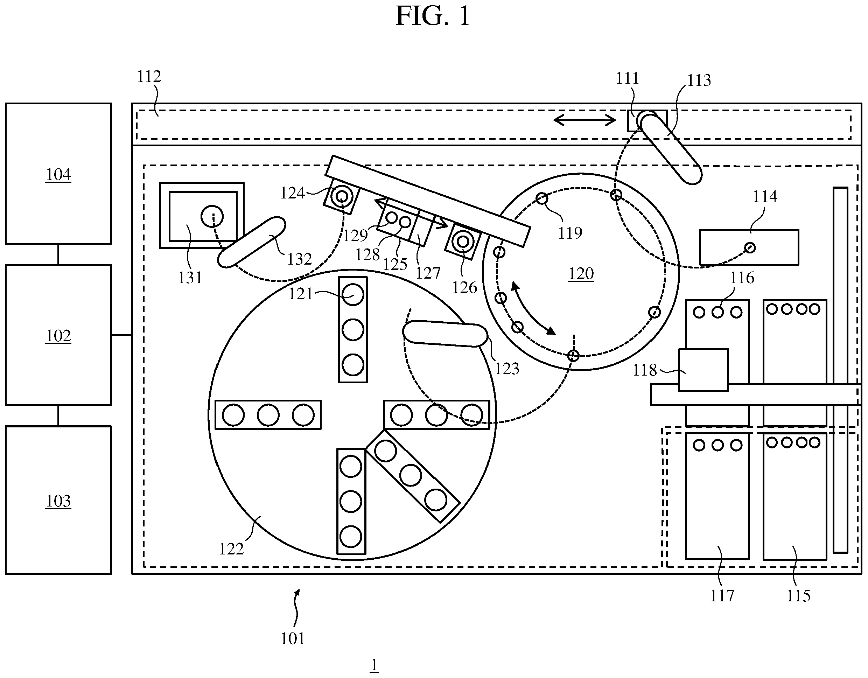

FIG. 1 is a schematic diagram of an automated analyzer 1 according to the present disclosure. The automated analyzer 1 includes an analysis unit 101 for performing an analysis operation, a control unit 102 for controlling an operation of an entire device, an input unit 103 for a user to input information to the device, and a display unit 104 for displaying information to the user. The input unit 103 and the display unit 104 may be the same, and a touch-panel type monitor is one example thereof. Further, the control unit 102 is a Central Processing Unit (CPU), for example, that reads and executes a program for controlling an amount of a washing liquid to be discharged.

The analysis unit 101 includes a first transport mechanism 112 for transporting a sample container 111 containing a sample to a sample collection position, a sample dispensing mechanism 113 for discharging the sample, a dispensing tip attaching and detaching section 114 for attaching and detaching a disposable dispensing tip for the sample dispensing mechanism 113 to the sample dispensing mechanism 113, a dispensing tip mounting rack 115 on which the dispensing tip is mounted, a reaction vessel mounting rack 117 on which a reaction vessel 116 is mounted, a second transport mechanism 118 for transporting the dispensing tip and the reaction vessel 116, a reaction vessel disk 120 capable of holding a liquid in the reaction vessel 116 at a constant temperature and having a plurality of openings 119, a reagent disk 122 for holding a reagent container 121 containing a measurement reagent, a reagent dispensing mechanism 123 for discharging the measurement reagent into the reaction vessel 116, a magnetic separation device 124 provided with magnets for capturing magnetic beads in the reaction vessel 116 to an inner wall of the reaction vessel 116, a stirring mechanism 126 that stirs the liquid contained in the reaction vessel 116 in a non-contact manner, a transporting and aspirating-discharging mechanism 125 that transports the reaction vessel 116 between the disk 120, the magnetic separation device 124, and the stirring mechanism 126, and that can aspirate and discharge a solution in the reaction vessel 116, a detector 131 that detects components in blood, and a dispensing mechanism for detector 132 for aspirating the components in the blood extracted from the reaction vessel 116 and discharging the components to the detector 131.

An outline of an analysis process of the automated analyzer 1 will be described below with reference to FIG. 1. Before the analysis, the automated analyzer 1 transports the reaction vessel 116 from the reaction vessel mounting rack 117 and disposes the reaction vessel 116 in the openings 119 on the reaction vessel disk 120.

The sample dispensing mechanism 113 accesses the dispensing tip attaching and detaching section 114 such that the dispensing tip can be attached to a tip before dispensing the sample. The sample dispensing mechanism 113 aspirates the sample from the sample container 111 via the dispensing tip and discharges the sample to the reaction vessel 116 on the reaction vessel disk 120. When a sample dispensing from one sample container 111 is completed, the sample dispensing mechanism 113 discards the dispensing tip to the dispensing tip attaching and detaching section 114.

The reagent dispensing mechanism 123 aspirates the measurement reagent from the reagent container 121 containing the magnetic beads on the reagent disk 122 and discharges the measurement reagent to the reaction vessel 116 on the reaction vessel disk 120. The reaction vessel disk 120 functions as, for example, an incubator, and incubates the reaction vessel 116 disposed in the openings 119 for a predetermined time.

A reaction proceeds due to the incubation of a certain period of time, and a substance to be measured and the magnetic beads are bound in the reaction vessel 116. Thereafter, the automated analyzer 1 performs a washing process and an elution process in order to improve the analysis accuracy. The expression "the substance to be measured and the magnetic beads are bound" means that, for example, a non-labeled antibody bound to the magnetic beads and the substance to be measured are bound by an antigen-antibody reaction.

### [Extraction of substance to be measured]

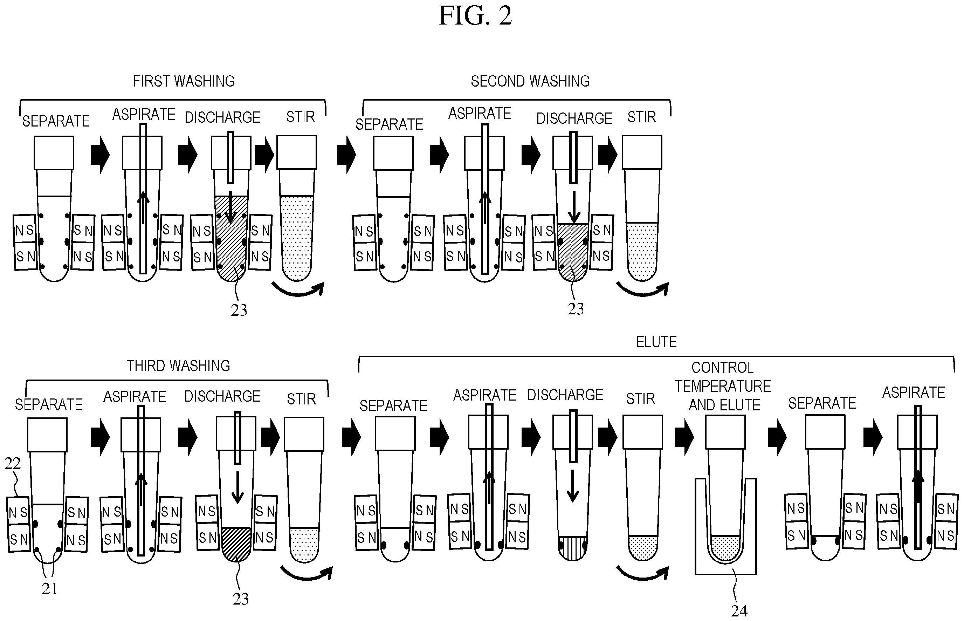

FIG. 2 is a schematic diagram showing a flow of a process for extracting the substance to be measured contained in the sample. In order to extract the substance to be measured from the sample, the automated analyzer 1 performs the washing process and the elution process. As shown in FIG. 2, in the present embodiment, the washing process is performed three times to wash and remove coexisting substances floating in the solution without binding to magnetic beads 21. The automated analyzer 1 sequentially reduces an amount of a washing liquid 23 to be injected in each of the three washing processes. For example, an amount of a washing liquid 23 for a first time is 250 µL, an amount of the washing liquid 23 for a second time is 160 µL, and an amount of the washing liquid 23 for a third time is 80 µL. In the elution process, the substance to be measured is eluted from the magnetic beads 21 by injecting 40 µL of eluate and controlling a temperature.

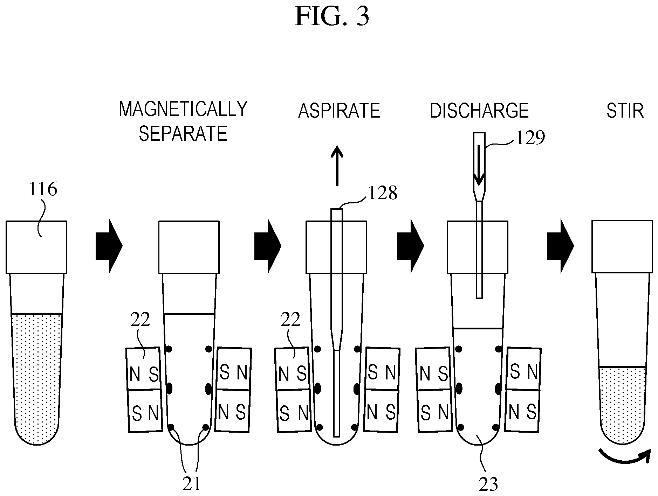

FIG. 3 is a diagram showing a first washing process. Hereinafter, the washing process will be described with reference to FIGS. 1 and 3.

The reaction vessel 116 containing the solution in which the magnetic beads 21 are suspended is transported to the magnetic separation device 124 by a gripping mechanism 127 of the transporting and aspirating-discharging mechanism 125. Magnets 22 are disposed around a recess of the magnetic separation device 124 into which the reaction vessel 116 is inserted, and the magnetic beads 21 are captured on the inner wall of the reaction vessel 116 by a magnetic field generated by the magnets 22. In an example shown in FIG. 3, the magnets 22 are stacked in two stages, and have a configuration in which the S pole of an upper magnet 22 faces the reaction vessel 116 and the N pole of a lower magnet 22 faces the reaction vessel 116. In this case, magnetic field intensity is high at both upper and lower edges of the magnets 22, so that the magnetic beads 21 are easily aggregated at both the upper and lower edges of the magnets 22. As described later, it is preferable that a height of the magnets 22 is a height in consideration of a height of a liquid surface of the solution to be injected into the reaction vessel 116. Further, the magnets 22 used for the magnetic separation device 124 are preferably neodymium-based magnets which are magnets having a high coercive force per unit volume from a viewpoint of a dimension. The magnets 22 may be electromagnets.

After supplementing the magnetic beads 21, the automated analyzer 1 uses an aspirating nozzle 128 of the transporting and aspirating-discharging mechanism 125 to remove the solution that does not include the magnetic beads 21 in the reaction vessel 116 by aspirating the solution with the aspirating nozzle 128. Subsequently, the automated analyzer 1 discharges the washing liquid 23 from a discharging nozzle 129 of the transporting and aspirating-discharging mechanism 125 to the reaction vessel 116. For example, the amount of the washing liquid to be discharged in the first washing process is adjusted such that the height of the liquid surface is at a position higher than the upper magnet 22 (a position where the magnetic field intensity is low). By adjusting the height of the liquid surface to the position where the magnetic field intensity is low, in a subsequent washing process, it is possible to prevent the case where the magnetic beads 21 aggregate near the liquid surface, the magnetic beads 21 are aspirated when aspirating the solution, and the solution is insufficiently aspirated due to a surface tension.

Thereafter, the reaction vessel 116 containing the magnetic beads 21 and the washing liquid 23 is transported to the stirring mechanism 126 by the gripping mechanism 127 of the transporting and aspirating-discharging mechanism 125. Since the magnetic beads 21 in the reaction vessel 116 transferred to the stirring mechanism 126 are not affected by the magnetic field, the magnetic beads 21 are isolated and re-suspended in the solution by being stirred by the stirring mechanism 126. Examples of a non-contact stirring mechanism 126 include a mechanism for applying a rotation operation combining rotation and revolution to the reaction vessel 116, that is, a mechanism that performs an eccentric stirring. When the non-contact stirring mechanism 126 is used, the sample or reagent is not taken out due to the solution adhering to a stirrer, so that the analysis accuracy is improved. After the magnetic beads 21 are re-suspended by the stirring mechanism 126, the reaction vessel 116 is transported to the magnetic separation device 124 again, and a second washing process is performed.

In the first embodiment, the automated analyzer 1 performs the above washing process three times. Here, in the second and subsequent washing processes, the amount of the washing liquid 23 to be discharged into the reaction vessel 116 is controlled to be smaller than an amount of the solution contained in the reaction vessel 116 before an aspirating operation, and therefore, the amount of the washing liquid 23 discharged at a second time is smaller than the amount of the washing liquid 23 discharged at a first time. Similarly, the amount of the washing liquid 23 discharged at a third time is smaller than the amount of the washing liquid 23 discharged at the second time. In addition, the amount of the washing liquid 23 discharged in each washing process is controlled such that a position of the liquid surface is the position where the magnetic field intensity is low, so that the position of the liquid surface of the washing liquid 23 discharged at the second time is adjusted such that the height of the liquid surface is positioned at a center of the upper magnet (the position where the magnetic field intensity is low), and the position of the liquid surface of the washing liquid 23 discharged at the third time is adjusted such that the height of liquid surface is positioned at a center of the lower magnet (the position where the magnetic field intensity is low). As described above, the washing process is performed by repeating magnetic separation and stirring a plurality of times, so that the coexisting substances removed.

The automated analyzer 1 according to the first embodiment can save the amount of the washing liquid 23 to be used by performing a plurality of washing processes in which the amount of the washing liquid 23 to be discharged is sequentially reduced as described above. In addition, the automated analyzer 1 of the first embodiment controls a discharge amount of the washing liquid 23 in each washing process such that the position of the liquid surface of the washing liquid 23 is the position where the magnetic field intensity is low, so that it is possible to prevent the case where the magnetic beads are aspirated when aspirating the solution, and the solution is insufficiently aspirated due to the surface tension.

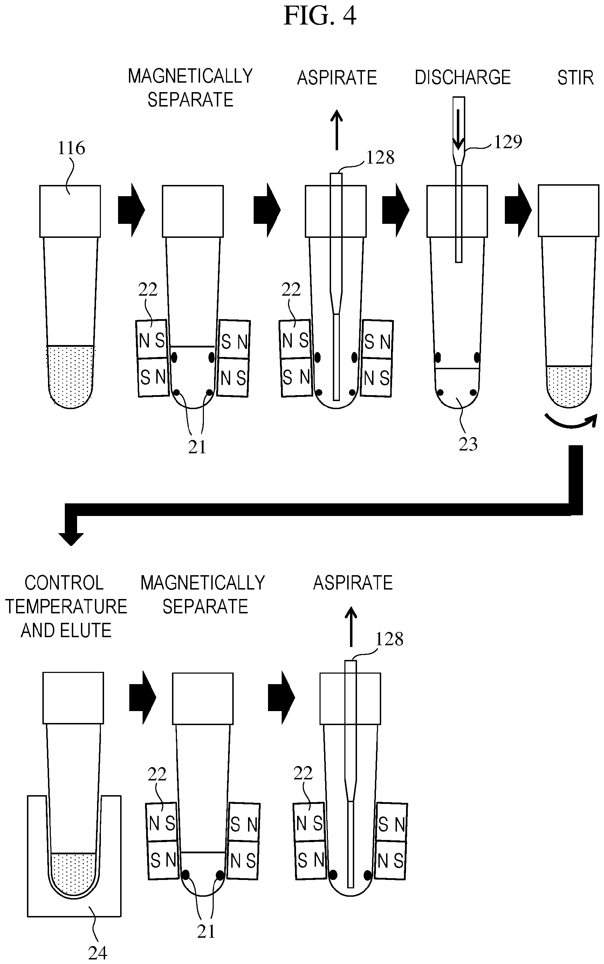

FIG. 4 is a schematic diagram showing a flow of the elution process. Hereinafter, the elution process will be described with reference to FIGS. 1, 2 and 4. FIG. 4 shows a flow after the third washing process is performed. After the third washing process is completed, the automated analyzer 1 magnetically separates the magnetic beads 21 again with the magnetic separation device 124, and aspirates the solution. Subsequently, the automated analyzer 1 discharges a smaller amount of the eluate than a reaction solution into the reaction vessel 116 and stirs the reaction vessel 116 with the stirring mechanism 126. Thereafter, the automated analyzer 1 transfers the reaction vessel 116 to the reaction vessel disk 120, controls the temperature of the reaction vessel 116 in an incubator 24 to promote a reaction, and elutes the substance to be measured from the magnetic beads 21. Then, by performing the magnetic separation again, a concentrated liquid containing the substance to be measured with the magnetic beads 21 being removed is created.

Subsequently, the automated analyzer 1 aspirates the concentrated liquid in the reaction vessel 116 on the magnetic separation device 124 by the dispensing mechanism for detector 132 and transports the concentrated liquid to the detector 131. The detector 131 includes a unit configured to detect an amount of light such as a photomultiplier tube, so as to measure the amount of the light emitted from the reaction solution (the concentrated liquid finally aspirated). Thereafter, the control unit 102 calculates a concentration value from light emission data using a calibration curve, and displays a calculated analysis result on the display unit 104.

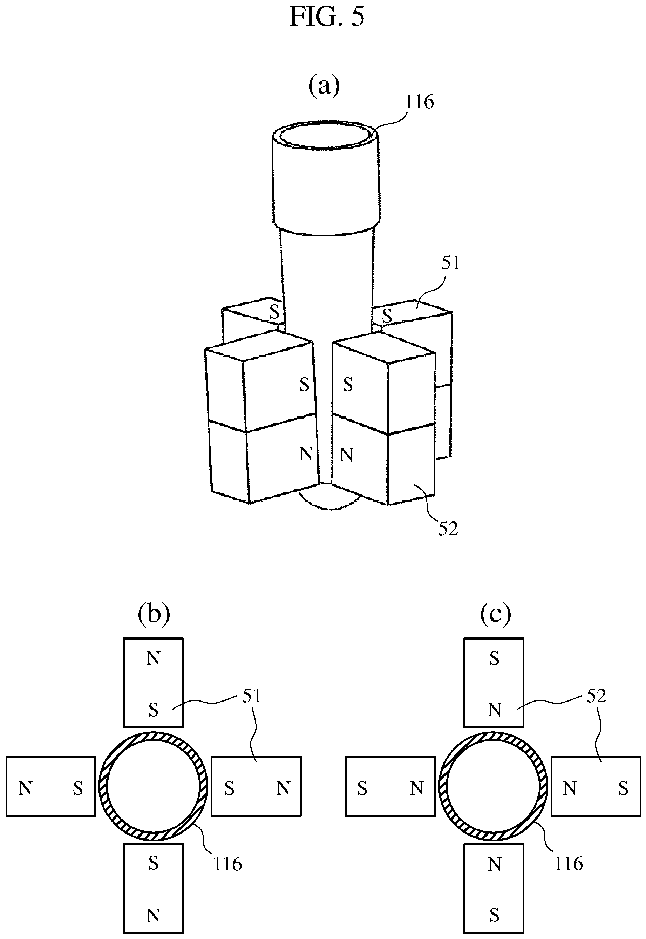

FIG. 5 shows a part of the magnetic separation device 124 according to the present embodiment. FIG. 5(a) shows a positional relationship between the reaction vessel 116 and the magnets 22. In an example shown in FIG. 5(a), the magnets 22 are disposed in two upper and lower stages. FIGS. 5(b) and 5(c) show plan views of the magnetic separation device 124, and magnet arrangements of a first stage (an upper stage) and a second stage (a lower stage) from a top are shown, respectively. In the present embodiment, an example in which the number of stages of the magnets 22 is two is shown, but the number of the stages of the magnets 22 may be three or more. Further, in the present embodiment, four magnets 22 are disposed in one stage, but an effect the same as in the present embodiment can be obtained as long as the amount of disposed magnets is an even number. For example, six or eight magnets 22 may be disposed in one stage. The height of the magnets 22 in each stage is the same, for example. Hereinafter, a reference numeral of the upper magnets is 51, and a reference numeral of the lower magnets is 52.

Four upper magnets 51 shown in FIG. 5(b) are disposed at equal intervals in a peripheral direction of the reaction vessel 116, with the S pole facing the center of the reaction vessel 116. On the other hand, four lower magnets 52 shown in FIG. 5(c) are disposed at the equal intervals in the peripheral direction of the reaction vessel 116, as the upper magnets 51, but the orientation of the magnetic pole is different from that of the upper magnets 51, that is, the N pole faces the center of the reaction vessel 116. In addition, in FIG. 5(a), all magnetic poles of the upper magnets 51 on a reaction vessel side are disposed as the S pole, and all the magnetic poles of the lower magnets 52 on the reaction vessel side are the N pole, but the magnetic poles of the upper magnets 52 may be the N pole, and the magnetic poles of the lower magnets 51 may be the S pole. That is, the magnets are arranged such that the magnetic pole of all magnets 22 in each stage facing the center of the reaction vessel 116 is the same, and the magnetic poles of magnets 22 disposed adjacently in the upper-lower direction are different from each other. In this way, a magnetic field distribution facilitating the supplementing of the magnetic beads 21 can be obtained.

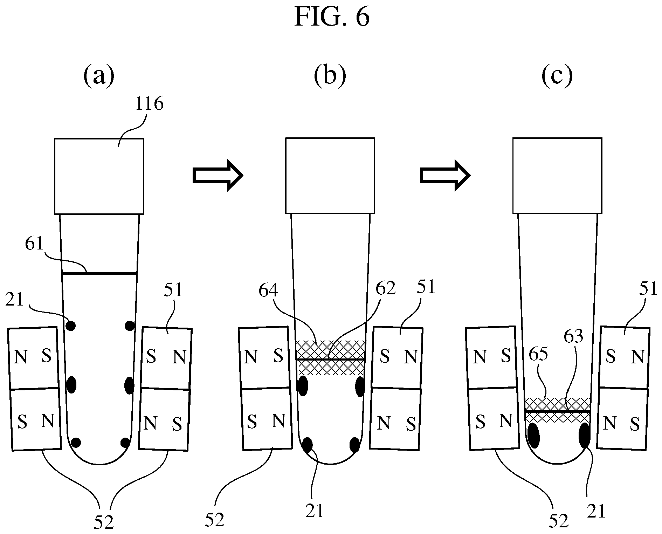

FIG. 6 shows states of capturing the magnetic beads 21 in the magnetic separation device 124. In a case of the magnet arrangements according to the present embodiment, strong magnetic fields are generated at both the upper and lower edges of the magnets 51 and 52, and therefore, the magnetic beads 21 have a feature of being captured at both edges of upper edges and lower edges of the magnets 51 and 52 as shown in FIG. 6(a). FIG. 6(a) shows a pattern in which the magnetic beads 21 are captured on the inner wall of the reaction vessel 116. As shown in FIG. 6(a), a liquid surface 61 of the washing liquid 23 in the first washing process is set higher than the upper magnets 51. In addition, as shown in FIG. 6(b), a liquid surface 62 of the washing liquid 23 in the second washing process is set near the center of the upper magnets 51. Further, as shown in FIG. 6(c), a liquid surface 63 of the washing liquid 23 in the third washing process is set near the center of the lower magnets 52. In the second and subsequent washing processes of the present embodiment, the liquid surface of the washing liquid 23 is set near the center of the magnets 51 and 52, and appropriate liquid surface ranges 64 and 65 are shown in a mesh pattern in FIGS. 6(b) and 6(c). A position of the mesh is a portion where the magnetic field intensity generated by the magnet arrangements of the present embodiment is low and does not overlap with a portion where the magnetic beads 21 aggregate. The position of the liquid surface may be a position where the mesh is provided. As described above, since the liquid surface and a position where the magnetic field intensity is high (a position where the magnetic beads 21 are easily collected) do not overlap, the magnetic beads 21 do not aggregate near the liquid surface.

According to the present embodiment, the magnetic beads 21 are always captured below the liquid surface, and the magnetic beads 21 do not aggregate on the liquid surface during the washing process in which a liquid amount is reduced. As a result, according to the automated analyzer 1, deterioration in the efficiency of the washing processes for removing impurities can be prevented, and a highly accurate measurement can be performed.

### <Second Embodiment

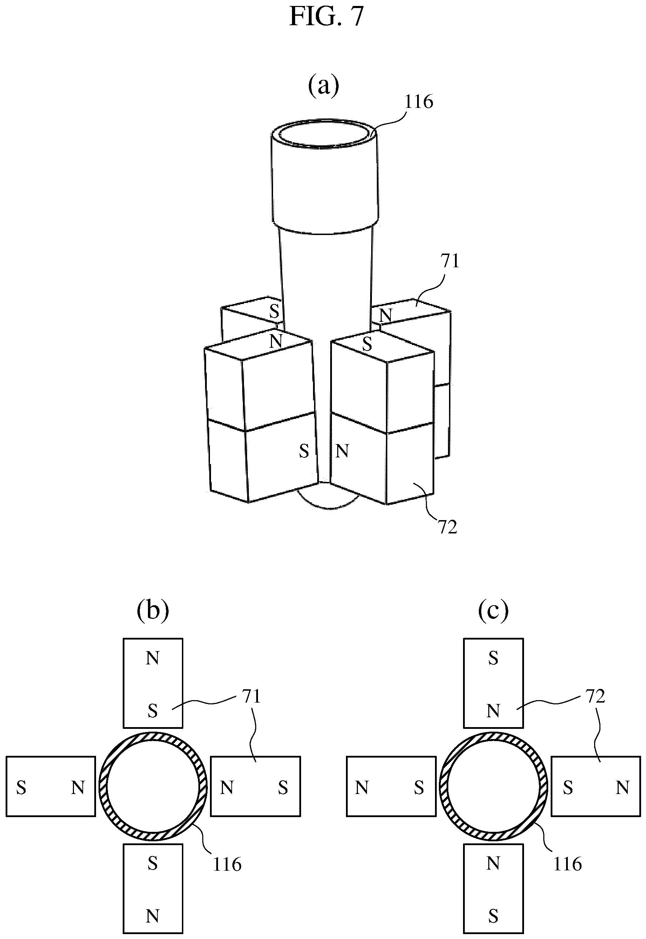

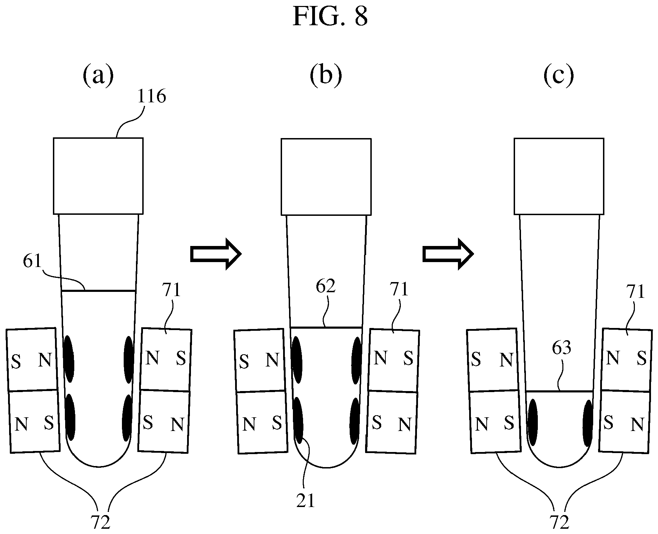

Next, the second embodiment will be described with reference to FIGS. 7 and 8. An automated analyzer according to the second embodiment is different from the automated analyzer 1 according to the first embodiment in the arrangement of the magnets 22 and a discharge control of the solution in the magnetic separation device. In FIGS. 7 and 8, components having the same reference numerals as those in FIGS. 1 to 6 indicate the same parts, and a repetitive description will be omitted. In the first embodiment, the upper magnets 51 are disposed at the equal intervals in the peripheral direction of the reaction vessel 116, with the S pols facing the center of the reaction vessel 116. On the other hand, according to the second embodiment, the magnets 22 of each stage are arranged such that, two magnets 22 facing each other have the same pole facing the center of the reaction vessel 116, and two adjacent magnets have poles different from each other facing the center of the reaction vessel 116. That is, the S pole and the N pole are alternately disposed along a periphery of the reaction vessel 116.

FIG. 7 shows positions of a magnetic separation device according to a second embodiment of the present disclosure. FIG. 7(a) shows the positional relationship between the reaction vessel 116 and two-stage magnets 22 disposed in the magnetic separation device. FIGS. 7(b) and 7(c) show the plan views of the magnetic separation device, and the magnet arrangements of the upper stage and the lower stage are shown, respectively. In the present embodiment, the number of the stages of the magnets 22 is shown as two as an example, but three or more stages may be provided. Further, in the present embodiment, as for the magnets 22, four magnets 22 are disposed in one stage, but the effect the same as in the present embodiment can be obtained as long as the amount of disposed magnets is an even number.

First-stage (upper stage) magnets 71 from a top view as shown in FIG. 7(b) are disposed at the equal intervals in the peripheral direction of the reaction vessel 116, in which two magnets 22 facing each other have the same pole facing the center of the reaction vessel 116, and two adjacent magnets 22 have different poles facing the center of the reaction vessel 116. On the other hand, second-stage (lower stage) magnets 72 from the top view as shown in FIG. 7(c) are disposed at the equal intervals in the peripheral direction of the reaction vessel 116, in which two magnets 22 facing each other have the same pole facing the center of the reaction vessel 116, and two adjacent magnets 22 have poles different from each other facing the center of the reaction vessel 116. Further, the magnets are arranged such that the magnetic poles of the magnets 22 disposed adjacently in the upper-lower direction are different from each other.

FIG. 8 shows states of capturing the magnetic beads 21 in the magnetic separation device according to the second embodiment. In a case of the magnet arrangements according to the second embodiment, since the strong magnetic fields are generated near the center of the magnets 71 and 72, the magnetic beads 21 are captured near the center of the magnets 71 and 72. FIG. 8(a) shows a distribution pattern of the magnetic beads 21 when the magnetic beads 21 are captured on the inner wall of the reaction vessel 116 in the first washing process. As shown in FIG. 8(a), in the first washing process, the liquid surface 61 of the washing liquid is set to a position higher than the upper magnets 71. FIG. 8(b) shows the distribution pattern of the magnetic beads 21 when the magnetic beads 21 are captured on the inner wall of the reaction vessel 116 in the second washing process. As shown in FIG. 8(b), in the second washing process, the liquid surface 62 of the washing liquid is set to a position near the upper edges of the upper magnets 71. Further, as shown in FIG. 8(c), the liquid surface 63 of the washing liquid is set near a portion between the upper magnets 71 and the lower magnets 72 in the third washing process. That is, according to the magnet arrangements of the second embodiment, the distribution pattern of the magnetic beads 21 generated when the magnetic beads 21 are captured on the inner wall of the reaction vessel 116 may be set so as not to overlap with the liquid surface. In other words, in each washing process, the automated analyzer controls the discharge amount of the washing liquid such that the height of the liquid surface is at the position where the magnetic field intensity is low.

According to the present embodiment, the magnetic beads 21 are always captured below the liquid surface, and the magnetic beads 21 do not aggregate on the liquid surface during the washing process in which the liquid amount is reduced. As a result, deterioration in the efficiency of the washing processes for removing impurities can be prevented, and a highly efficient automated analyzer can be obtained.

### <Third Embodiment>

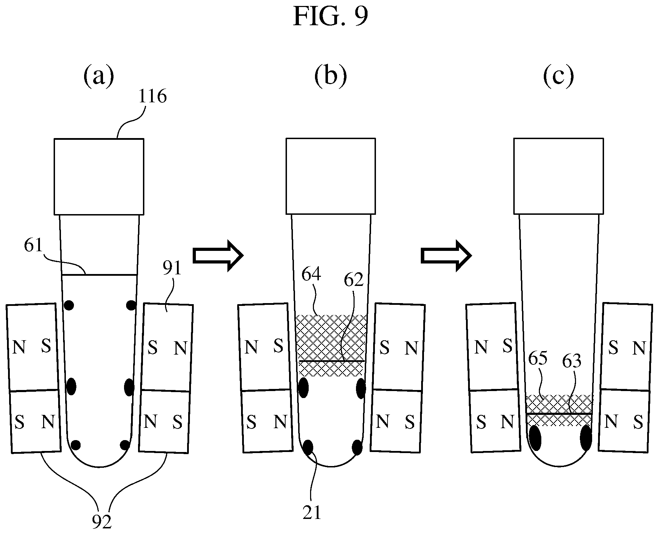

In the first embodiment and the second embodiment, heights of the magnets 22 in each of the upper and lower stages are the same. However, the heights of the magnets 22 may be different at each stage. FIG. 9 shows magnet arrangements according to the third embodiment. In the magnetic separation device 124 shown in the third embodiment, the height of the upper magnets 91 is higher than the height of the lower magnets 92. Even in such a case, the fact that the magnetic field intensity is high at both upper and lower edges of the magnets is the same as above, so that the magnetic beads 21 are captured at both upper and lower edges of the magnets in each stage. Therefore, as shown in FIG. 9, a distance between positions where the magnetic beads 21 are densely captured differs depending on the height of the magnets. In the case of the magnet arrangements as shown in FIG. 9, the liquid surface range 64 of the washing liquid 23 (a mesh pattern portion in the figure) in the second washing process can be made larger as compared with that in the first embodiment. In this way, by making the heights of the magnets it each stage different from each other, instead of being the same, it is possible to widen an applicable range of the discharge amount of the washing liquid 23 in the washing process. The liquid surface range 65 of the washing liquid 23 in the third washing process is the same as that in the first embodiment.

In the first to third embodiments described above, the positions of the liquid surfaces 61, 62, and 63 of the washing liquid are defined based on, for example, a position where an inner wall surface of the reaction vessel 116 is in contact with the washing liquid in consideration of an influence of a meniscus force. For example, when a contact angle is small, that is, when the liquid surface is a concave, the position where the inner wall surface of the reaction vessel 116 is in contact with the washing liquid is higher than the center of the liquid surface. In addition, when the contact angle is big, that is, when the liquid surface is a convex, the position where the inner wall surface of the reaction vessel 116 is in contact with the washing liquid is lower than the center of the liquid surface.

<Modification>

In the first to third embodiments, the magnets 22 are disposed in two upper and lower stages. However, the magnets 22 may be disposed in only one stage. In this case, as for the magnets 22, for example, all magnets 22 have the same pole facing the reaction vessel 116, and are disposed at the equal intervals around the reaction vessel 116. That is, the magnets 22 are disposed such that the magnetic field intensity is high at both upper and lower edges of the magnets 22. Alternatively, the magnets 22 may be disposed so as to have a magnetization pattern the same as that in the first to third embodiments. In this case, the automated analyzer adjusts the amount of the washing liquid 23 in the first washing process such that the position of liquid surface is higher than the upper edges of the magnets 22, and adjusts the amount of the washing liquid 23 in the second washing process such that the liquid surface is positioned in the center of the magnets 22.

The invention is not limited to the embodiments described above and includes various modifications. For example, the embodiments described above have been described in detail for easy understanding of the invention, and the invention is not necessarily limited to those including all the configurations described above. In addition, a part of the configuration of one embodiment can be replaced with the configuration of another embodiment, and the configuration of another embodiment can be added to the configuration of one embodiment. In addition, a part of the configuration of each embodiment may be added, deleted, or replaced with another configuration.

### Reference Sign List

- 1:: automated analyzer
- 101:: analysis unit
- 102:: control unit
- 103:: input unit
- 104:: display unit
- 111:: sample container
- 112:: first transport mechanism
- 113:: sample dispensing mechanism
- 114:: dispensing tip attaching and detaching section
- 115:: dispensing tip mounting rack
- 116:: reaction vessel
- 117:: reaction vessel mounting rack
- 118:: second transport mechanism
- 119:: opening on reaction vessel disk
- 120:: reaction vessel disk
- 121:: reagent container for measurement
- 122:: reagent disk
- 123:: reagent dispensing mechanism
- 124:: magnetic separation device
- 125:: transporting and aspirating-discharging mechanism
- 126:: stirring mechanism
- 127:: gripping mechanism
- 128:: aspirating nozzle
- 129:: discharging nozzle
- 131:: detector
- 132:: dispensing mechanism for detector
- 21:: magnetic beads
- 22:: magnet
- 23:: washing liquid
- 24:: incubator
- 51:: first-stage magnet
- 52:: second-stage magnet
- 61 to 63:: liquid surface
- 64 and 65:: applicable liquid surface range

## Claims

1. A magnetic separation method comprising:
a plurality of washing processes for separating a magnetic substance and a nonmagnetic substance using a magnetic separation device and a stirring mechanism, wherein
the plurality of washing processes includes at least a first washing process and a second washing process,
the first washing process includes:
a step of inserting a reaction vessel containing a solution including the magnetic substance and the nonmagnetic substance into a recess provided in the magnetic separation device and capturing the magnetic substance using a plurality of magnets that are each disposed along a peripheral direction of the recess such that the same pole faces the reaction vessel;
a step of aspirating the solution with the magnetic substance being captured;
a step of discharging liquid to the reaction vessel such that a surface of the liquid goes to a position higher than upper edges of the magnets; and
a step of removing the reaction vessel from the magnetic separation device and stirring the liquid held by the reaction vessel using the stirring mechanism, and
the second washing process includes:
a step of inserting the reaction vessel into the magnetic separation device and aspirating the liquid with the magnetic substance being captured;
a step of discharging the liquid to the reaction vessel such that a surface of the liquid goes to a position lower than the upper edges of the magnets where magnetic field intensity is lower than that at a position of the upper edges of the magnets; and
a step of removing the reaction vessel from the magnetic separation device and stirring the liquid held by the reaction vessel using the stirring mechanism.

2. The magnetic separation method according to claim 1, wherein
in the magnetic separation device,
the plurality of magnets are disposed in a configuration having a first stage and a second stage positioned below the first stage along a vertical direction of the recess, and
the first stage and the second stage each include an equal number of magnets, the magnets in the first stage and the magnets in the second stage are vertically adjacent to each other with different poles,
a position of the surface of the liquid in the first washing process is a position higher than upper edges of the magnets in the first stage, and
a position of the surface of the liquid in the second washing process is a position lower than the upper edges of the magnets in the first stage where the magnetic field intensity is lower as compared with that at the position of the upper edges of the magnets in the first stage.

3. The magnetic separation method according to claim 2, wherein
the position of the surface of the liquid in the second washing process is between the upper edges and lower edges of the magnets in the first stage.

4. The magnetic separation method according to claim 3, further comprising:
a third washing process, wherein
the third washing process includes:
a step of inserting the reaction vessel into the magnetic separation device and aspirating the liquid with the magnetic substance being captured;
a step of discharging liquid to the reaction vessel such that a surface of the liquid goes to a position lower than upper edges of the magnets in the second stage where the magnetic field intensity is lower as compared with that at the position of the upper edges of the magnets in the second stage; and
a step of removing the reaction vessel from the magnetic separation device and stirring the liquid held by the reaction vessel.

5. The magnetic separation method according to claim 4, wherein
a position of the surface of the liquid in the third washing process is between the upper edges and lower edges of the magnets in the second stage.

6. An automated analyzer that performs the magnetic separation method according to claim 1.

7. A magnetic separation method comprising:
a plurality of washing processes for separating a magnetic substance and a nonmagnetic substance using a magnetic separation device and a stirring mechanism, wherein
the plurality of washing processes includes at least a first washing process and a second washing process,
the first washing process includes:
a step of inserting a reaction vessel containing a solution including the magnetic substance and the nonmagnetic substance into a recess provided in the magnetic separation device and capturing the magnetic substance using a plurality of magnets, the magnets being disposed such that a first stage and a second stage positioned below the first stage along a vertical direction of the recess are each provided with an equal number of magnets, the magnets in the first stage and the magnets in the second stage are vertically adjacent to each other with different poles, two adjacent magnets in the first stage have poles different from each other facing the reaction vessel, and two magnets facing each other have the same pole facing the reaction vessel;
a step of aspirating the solution with the magnetic substance being captured;
a step of discharging liquid to the reaction vessel such that a surface of the liquid goes to a position higher than upper edges of the magnets; and
a step of removing the reaction vessel from the magnetic separation device and stirring the liquid held by the reaction vessel using the stirring mechanism, and
the second washing process includes:
a step of inserting the reaction vessel into the magnetic separation device and aspirating the liquid with the magnetic substance being captured;
a step of discharging liquid to the reaction vessel such that a surface of the liquid goes to a position lower than a position higher than the upper edges of the magnets in the first stage where magnetic field intensity is lower as compared with that at a center of the magnets in the first stage or the magnets in the second stage; and
a step of removing the reaction vessel from the magnetic separation device and stirring the liquid held by the reaction vessel using the stirring mechanism.

8. The magnetic separation method according to claim 7, wherein
the position of the surface of the liquid in the second washing process is a position of the upper edges or lower edges of the magnets in the first stage.

9. The magnetic separation method according to claim 7, further comprising
a third washing process, wherein
the position of the surface of the liquid in the second washing process is the position of the upper edges of the magnets in the first stage, and
the third washing process includes:
a step of inserting the reaction vessel into the magnetic separation device and aspirating the liquid with the magnetic substance being captured;
a step of discharging liquid to the reaction vessel such that a surface of the liquid goes to a position of the lower edges of the magnets in the first stage or the upper edges of the magnets in the second stage; and
a step of removing the reaction vessel from the magnetic separation device and stirring the liquid held by the reaction vessel.

10. An automated analyzer that performs the magnetic separation method according to claim 7.
